# EUROPEAN PATENT APPLICATION

(11) **EP 1 410 774 A1**
(43) Date of publication of application: **21.04.2004**
(21) Application number: 02447201.1
(22) Date of filing: 18.10.2002
(51) Int. Cl.: A61F 5/01, A61H 1/00, A61H 1/02

(54) **Orthotic device with detachable motor unit**

(71) Applicant: Tob NV, 9051 Sint-Denijs-Westrem (BE)
(72) Inventor: The designation of the inventor has not yet been filed
(74) Representative: Brants, Johan Philippe Emile

(57) **Abstract**

The present invention relates to an individually tailored orthotic brace with detachable motor unit. The invention concerns a rehabilitation device comprising a brace able to embrace a jointed limb, and a detachable motor unit. The motor unit can be temporarily attached to the brace by means of connector elements provided on the hinge of said brace. The motor unit is programmed to impose movements to the embraced limb and/or to register the movements performed by the embraced limb. The present invention also relates to a method for the rehabilitation of an injured limb by using the rehabilitation device according to the invention.

## Description

### FIELD OF THE INVENTION

The present invention is related to the field of orthotics and rehabilitation. The present invention relates to a rehabilitation device comprising an individual orthotic device more in particular a brace with a detachable motor unit. The invention also relates to a method for the rehabilitation of an injured limb using said rehabilitation device.

### BACKGROUND OF THE INVENTION

Each year thousands of individuals face the need to perform some type of rehabilitation therapy program such as physical therapy or occupational therapy. Traditionally, rehabilitation therapy for a patient involves diagnosing the impairment, disability, or handicap, evaluating the individual's capabilities and ambitions, establishing a rehabilitation program directed toward those goals, and performing the rehabilitation program. Two major tasks of successful rehabilitation therapy are overcoming the individual's lack of motivation and evaluating the individual's progress in the rehabilitation program.

When a limb is forced into inactivity because of injury or surgery, passive motion applied continuously can help reduce joint stiffening and muscle atrophy. Both muscles and bones should be exercised to maintain strength. When a joint is immobilized for a period of time, such as when a patient's limb is immobilized in a cast or splint in order to allow a broken bone to heal, connected tissue including ligaments, tendons, joint capsules, and other related structures, at the joint tends to shorten, resulting in a decreased range of motion at the joint. This condition is exacerbated based on the length of time that the joint is immobilized.

Brace-like devices for immobilizing and supporting an injured limb during the healing process have been reported. Such immobilizing braces generally have the advantage of being light and are individually tailored. This individual brace is worn until complete recovery of the limb.

In the recovery process, it is often necessary to exercise the injured limb. Specific training equipment is used for this purpose. Motorized equipment for exercising immobilized limbs can impose a dynamic force to the limb of a patient. One type of such exercise equipment provides to attach the exercise equipment to a limb support structure or to a fastening means that is part of the equipment. This type of equipment attaches to the limb segments to permit motion of the limb segments, such as for example, motion of the leg with respect to the thigh about the knee, the thigh and trunk about the hip, the arm and trunk about the shoulder and the forearm and arm about the elbow. An example of such exercising equipment is provided in US 5,891,061, which describes a motorized brace for applying a dynamic force to a jointed limb of a patient.

However, the limb support structures or fastening means used in such equipment are not individually tailored and need to be adjusted to a patient. Also, such motorized equipment is cumbersome and complicated to use. Because these limb support have to be adjusted to the patient by specialized personnel such as physiotherapists or physicians structures, and because the equipment is cumbersome and complicated to use, patients in need of a rehabilitation treatment have to go to a hospital or a rehabilitation center for using the orthotic devices and performing their prescribed motion exercises.

In view of the major drawbacks of the above-described training equipment there remains a need in the art for providing lighter motorized equipment, which can be individually tailored. The present invention provides a solution to this and other problems, and offers other advantages over the prior art.

In particular, it is an object of the present invention to provide a motorized orthotic device, which is light and individually tailored.

The present invention also aims to provide a user-friendly orthotic device, which overcomes the need to go to a hospital or a clinic for performing the prescribed exercises.

Another object of the invention is to provide a lighter motorized orthotic device, which enables to perform a prescribed schedule of repetitive movement exercises.

Another object of the invention is to provide a lighter motorized orthotic device which enables to control whether a patient using the device has performed all repetitive movement exercises.

### SUMMARY OF THE INVENTION

The present invention relates to a rehabilitation device and to a method for the rehabilitation of an injured body part comprising an articulation using said device. As used herein the term "orthotic device" and "rehabilitation device" are used as synonyms and refer to the device according to the invention enabling the rehabilitation of an injured body part comprising an articulation. The term "rehabilitation" as used herein refers to the process of reinforcing muscles and/or exercising movements of body parts comprising an articulation or joint movements after injury or chirurgical operations.

The present invention relates to an orthotic device, more in particular a brace for supporting or supplementing weakened or abnormal body parts comprising an articulation. Examples of said body parts are limbs such as an arm, a leg, a foot, etc., the articulations of these limbs being an elbow, a knee, an ankle etc. Other possible body parts comprising an articulation may be neck, shoulder, back, collar, etc.

In a first aspect, the present invention relates to a device, for example a brace able to partly embrace and support a body part comprising an articulation, said device being connectable to a detachable motor unit and comprising
- a first device portion for supporting the portion of said body part provided on the upper side of a joint in said body part,
- a second device portion for supporting the portion of a body part comprising an articulation provided on the lower side of said joint in the body part,
- a hinge for connecting said first device portion with said second device portion, said hinge being provided with connector elements for temporarily attaching a detachable motor unit able to control the hinge,
whereby said connector elements are provided at the joint of the body part for temporarily attaching a detachable motor unit.

In a second aspect, the invention relates to a motor unit being temporarily attachable to the brace according to the invention by means of connector elements provided on the hinge of said brace, said detachable motor unit being able to control the hinge of said brace. The motor unit according to the invention is programmable for imposing movements to a body part comprising an articulation and/or for registering the movements performed by a body part comprising an articulation.

In a third aspect the present invention relates to a rehabilitation device comprising the above-described brace whereon the above-described programmable motor unit is temporarily attached. The brace according to the invention is provided with connector elements on the hinge of said brace. The motor unit is attached to the brace by means of these connector elements provided. According to the invention the motor unit is directly connected on the connector elements of the brace and not on the portion of the brace supporting the body parts comprising an articulation. This type of connection is highly effective, since it enables to induce joint-specific motions to the embraced body parts comprising an articulation.

In a fourth aspect the present invention relates to a method for the rehabilitation of an injured body part comprising an articulation using the above-described rehabilitation device, i.e. the brace according to the invention connected to the motor unit according to the invention.

Other benefits and advantages of the invention will become apparent upon reading and understanding the below given specification and accompanying drawings.

### DETAILED DESCRIPTION OF THE DRAWINGS

Figure 1 shows a perspective view of the brace according to the invention comprising a first and a second brace portion, which are connected by a hinge. The hinge is represented without connector elements.

Figure 2 shows a perspective view of the brace according to the invention comprising a first and a second brace portion, which are connected by a hinge. The hinge is represented with the connector elements for temporarily connecting a detachable motor unit able to control the hinge.

Figure 3 shows a perspective view of the rehabilitation device according to the invention, comprising a brace and a motor unit connected thereto.

Figure 4 represents a cross-sectional view through the horizontal hinging plane of the brace.

Figure 5 represents the connection of the brace provided with the motor unit to a control panel.

The foregoing summary, as well as the following detailed description of preferred embodiments of the invention, will be better understood when read in conjunction with the appended drawings. The drawings are for the purpose of illustrating the present invention, which is not limited to the devices and instrumentalities shown.

### DETAILED DESCRIPTION

The invention relates to a new and improved apparatus and method for rehabilitation of injured body parts comprising an articulation. The description of a preferred embodiment is, non limitative, directed to a limb in the present description. The invention also provides a new and improved apparatus and method for coding rehabilitation treatment and recording the progress of the rehabilitation.

In a first embodiment, the invention relates to a brace able to embrace a jointed limb, said brace being connectable to a detachable motor unit and comprising
- a first brace portion for supporting the portion of said limb provided on the upper side of a joint in said limb,
- a second brace portion for supporting the portion of a limb provided on the lower side of said joint in the limb,
- a hinge for connecting said first brace portion with said second brace portion, said hinge being provided with connector elements for temporarily attaching a detachable motor unit able to control the hinge,
whereby said connector elements are provided at the limb joint for temporarily attaching a detachable motor unit.

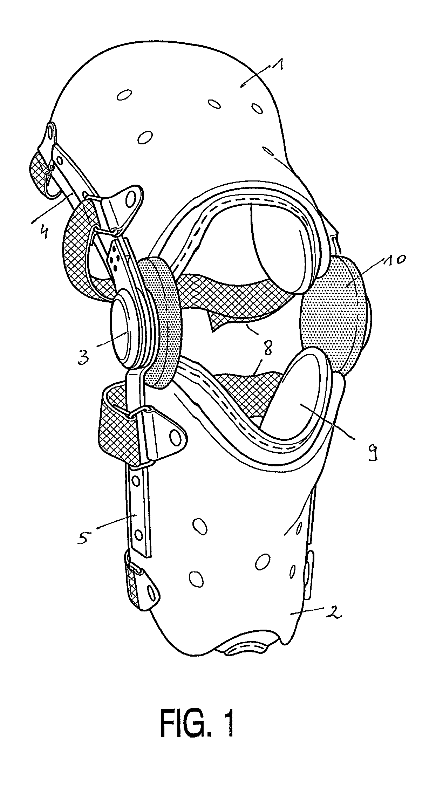

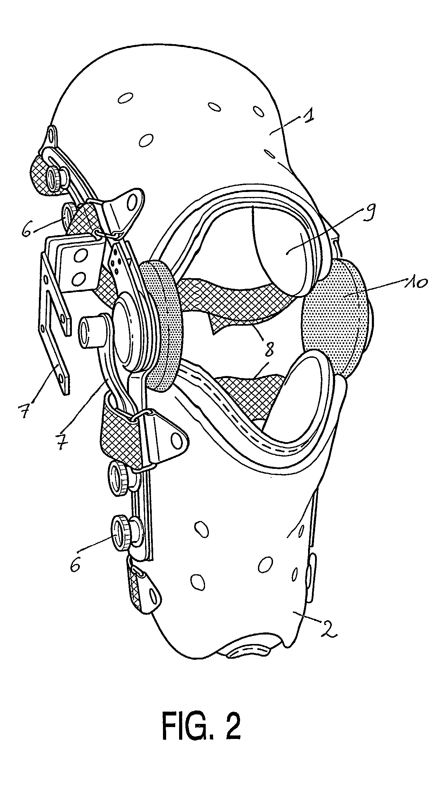

Figures 1 and 2 show a perspective view of the brace according to the invention comprising a first brace portion **1** and a second brace portion **2**, which are connected by a pivotable connection, which preferably is a hinge **3**.

An important element improving the brace's comfort from a patient's point of view comprises the fact that the brace according to the invention is "individually tailored". This means that the brace is fabricated to fit the limb anatomy of an individual person.

In a preferred embodiment the first and second brace portion of the brace are provided with fastening means. Preferably, the first and second brace portions are attached to the patient's limb using straps **8**, which are connected together with VELCRO™-textile fastening material or a similar releasable fastening material to allow a patient to detachably attach the first and second brace portions in position. Such fastening means provides an easy and comfortable application of the brace to a patient's limb.

Additionally, cushioned inserts **9** can be provided between the first and second brace portions and the patient's limb in order to prevent chafing and to improve the patient's comfort. Preferably, the first and second brace portions extend on both sides of the patient's limb and the pivotal connection preferably only on one side of the joint. However, it will be recognized by those skilled in the art from the present disclosure that the pivotal connection could also be provided on either side of the joint to provide better stability, if desired. Furthermore, cushioned inserts **10** can also be provided between the hinge and the patient's joint, in order to prevent chafing or friction of the brace hinge to the limb joint of the patient and to improve the patient's comfort.

Preferably, the brace is meant for embracing a leg or an arm. In a preferred embodiment, the brace is adapted to be attached to a patient's arm with the first brace portion being connected to the forearm and the second brace portion being connected around the bicep and tricep area. The hinge between the first and second brace portions is aligned with the patient's elbow. In another preferred embodiment the brace is adapted to be attached to a patient's leg with the first brace portion being connected around the thigh-bone and the second brace portion being connected around the shinbone. However, it will be recognized by those skilled in the art from the present disclosure that the brace could also be applied on either side of a patient's wrist, or could be applied to a patient's leg around the knee joint or the ankle, if desired. The first and second brace portions would be sized for the specific application, with the hinge being aligned in each case with the joint. The brace could also be applied to animals.

A pivotable connection is provided between the first and second brace portions. The pivotable connection is adapted to be generally aligned with the joint in the patient's limb when the brace is attached to the patient's limb. According to a preferred embodiment of the invention the pivotable connection consists of a hinge **3**, as shown on figures 1 and 2. The brace remains open between the first and the second brace portions at the position of the joint in the patient's limb and the brace is attached to the patient's limb. As a consequence, the limb joint is not embraced and remains free, which greatly improves and fastens the recovery of the joint movement and of the joint muscles and of the connective tissue of the joint, including ligaments, tendons, joint capsules and other related structures.

The hinge **3** of the brace connects the first brace portion **1** with the second brace portion **2** and consists of an upper hinge leg **4** and a lower hinge leg **5**. The upper hinge leg is preferably attached to the first brace portions and the lower hinge leg is preferably attached to the second brace portions. A connection point **3** is provided between said upper and lower legs of the hinge. It will be understood by those skilled in the art from the present disclosure that the upper and a lower hinge leg of the hinge can rotate to one another in function of the movements of the patient's limb embraced in the brace portions. The hinge legs can be fastened on the respective brace portions by gluing. In a preferred embodiment, the hinge legs are fastened on the respective brace portions by means of bolts **6**, as represented on figure 2.

On the figures 1 and 2, the hinge is represented without and with connector elements **7**, respectively. The connector elements **7**, provided at the limb joint are meant for temporarily attaching a detachable motor unit. The connector elements may comprise but are not limited to protrusions, pin structures, cogwheel structures, magnetic connecting means, releasable fastening materials such as VELCRO™-textile fastening material or a similar material. In a preferred embodiment, as represented on figure 2, the connector elements consists of a plate-like structure and a pin.

The first **1** and second **2** portion of the brace are preferably made of a strong, lightweight material such as plastic or aluminum. Also, the hinge of the brace and the connector elements on said hinge are preferably made of a strong, lightweight material such as plastic or aluminum. By using strong lightweight material for making the brace portions as well as the hinge structure, the obtained brace as a whole remains light and thus easy to carry by a patient.

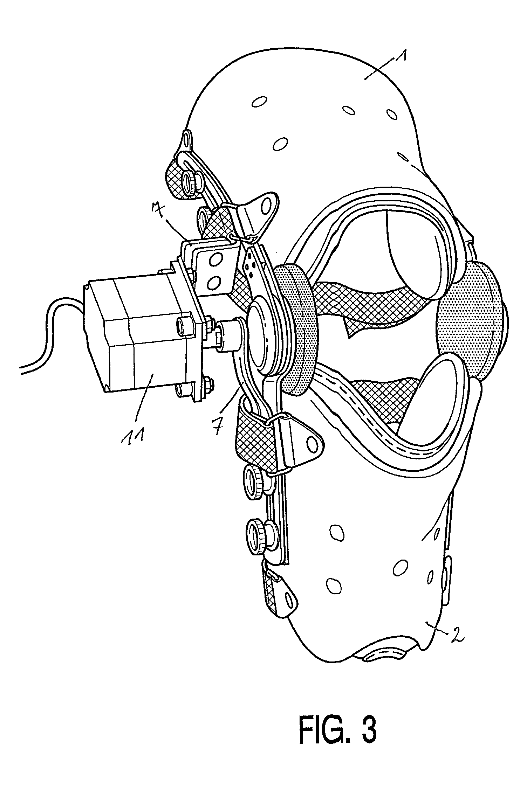

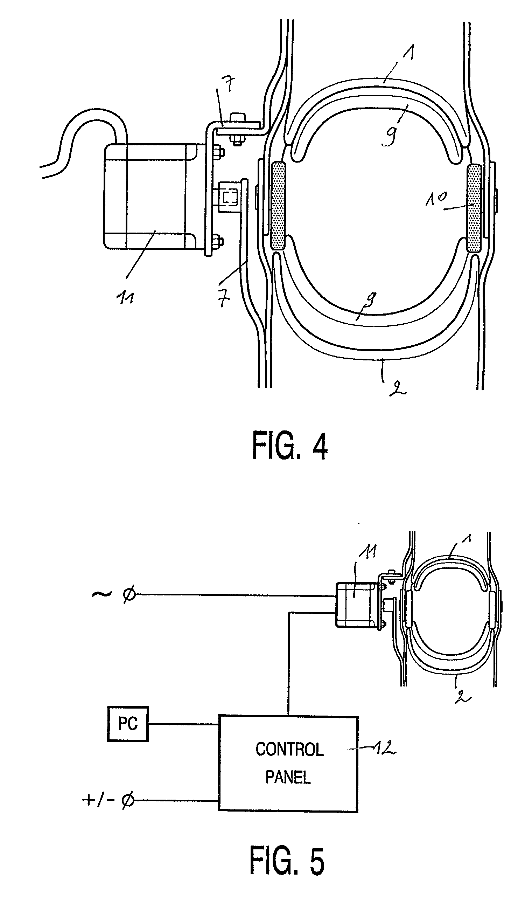

In another aspect the present invention relates to a motor unit **11** being temporarily attachable to the brace according the invention by means of connector elements **7** provided on the hinge **3** of said brace. Such connection is represented on figure 3 and 4. The motor unit **11** can be connected to the brace at the time a movement is to be imposed on the embraced limbs, and can be disconnected and detached from the brace, when the brace is meant to provide support of the limb only.

In a preferred embodiment the motor unit is made of a lightweight material. It will be recognized by those skilled in the art from the present disclosure that the motor unit could be made from suitable metallic or polymeric materials, such as steel, brass, PVC or any other material having the desired strength.

As represented on figure 5, the motor unit **11** according to the invention is connectable to a control panel **12,** said control panel being connectable to PC equipment and to a power-supplier. The control panel **12** of the motor unit may be connected to the electricity net by means of a cable with plug. Optionally, the motor unit **11** may also be provided with a direct connection to a power-supplier. For instance, the motor unit may be directly connected to the electricity net by means of a cable with plug, which can be plugged in a socket.
The detachable motor unit is able to control the hinge of said brace. The motor unit imparts a dynamic force on the brace. In another embodiment, the motor unit according to the invention can be programmed for imposing movements to a limb. The motor unit according to the invention is a motor unit wherein the type of movement, e.g. translational or rotation movements, the degrees of movements, and the movement frequency can be programmed.

When an individual sustains a physical injury which involves damage to bones, muscle tissue, connective tissue or the like, the physician treating the individual will make a determination as to whether exercise will be allowed. Taking the patient's treatment plan and its pain tolerance into consideration, the physician determines the appropriate schedule of repetitive movement exercises.

For many people, rehabilitation programs last several weeks, months, and even years. During rehabilitation, patients are taught how to move correctly and are given exercises to diminish their movement impairments. To correctly learn movements and exercises, patients often require many repetitions of instructions from the therapists. However, the repetition of exercises by a patient under continuous supervision of a therapist is prohibitively costly. Lack of motivation to continue with practice is detrimental to progress in a rehabilitation program. Successful rehabilitation depends not only on the patient's repetition of exercises and movements, but also requires that the exercises and movements be performed correctly. However, a major drawback of the orthotic devices known in the art providing continuous passive motion to a limb is their inability to allow control whether the patient using the device has performed all exercises prescribed by a physician. Using the braces currently known in the art, a physician cannot judge how well the patient has adhered to a schedule of repetitive exercise events. Unfortunately, however, the physician is generally unable to obtain adequate information or assurances about the manner in which a particular patient will conduct the prescribed exercises. Furthermore, the physician is unable to obtain adequate feedback after the patient performs any specific prescribed exercise. Also, the physician cannot judge the amount of stress the patient can or will exert voluntarily, and cannot estimate how well the patient will adhere to a schedule of repetitive exercise events.

The present invention provides a solution to this drawback by providing a orthotic device permitting to register the movements performed by the patient. The motor unit **11** according to the invention can be programmed for registering the movements performed by a limb. This feature enables a physician to control whether a patient has carried out all prescribed exercises and has adhered to a schedule of repetitive exercise events. It further permits the prescribing physician to evaluate the patient's progress in regard to the prescribed exercises. The motor unit is programmed in such a way as to keep a record of all performed exercises, so the physician can ensure the patient is complying with the prescribed exercise regime.

The element playing a crucial role in the programming of the motor unit consists of the control panel **12** of the motor unit. The method for programming the motor unit comprises the steps of connecting said motor unit with PC equipment via the control panel and loading a software program on said motor unit. Practically, as illustrated on figure 5 the control panel is connected to PC equipment and loaded with a software program designed for imposing a particular set of movements or motion exercises in function of a patients' need. In addition, the software program is also designed in such a way as to enable the registration of the performed exercises. The control panel of the motor unit can be connected to PC equipment either during or after the rehabilitation period of a patient, providing adequate information about the manner in which a particular patient conducts, or has conducted the prescribed exercise, respectively. The control panel provided on the motor unit provides adequate feedback information on any prescribed exercise that a patient has performed.

Additionally, via the control panel of the motor unit the motion exercises can be corrected, if necessary. Since the control panel gives a patient and/or a physician immediate feedback with regard of the prescribed exercise regime, the physician may decide to adapt the motion exercises. For example, progressive increases in range of motion may provide faster and earlier recovery of the injured limb.

The present invention also relates in another embodiment to the software program for programming a motor unit according to the above-described method. This software program involves a program for imposing movements to a limb and/or a program for registering the movements performed by said limb.

In a preferred embodiment the invention relates to a rehabilitation device comprising the above-described brace, wherein the above-described programmable motor unit is temporarily attached by means of the connector elements provided on the hinge of said brace.

Connection between the brace and the motor unit according to the invention occurs by means of the connector elements on the hinge of the brace. The motor unit is directly connected to the connector elements of the brace and not to the brace portions embracing the limb, as shown on figures 3 an 4. Such connection has the major advantage of such that joint-specific motions can be imposed to the embraced limbs. This kind of joint-specific motions greatly improves and fastens rehabilitation of the injured joint, and cannot be performed when using conventional braces or rehabilitation devices.

The connection between the brace and the motor unit further involves other advantages. One advantage of this type of connection is the fact that the motor unit can be easily detached when no movement needs to be imposed on the embraced limbs, or can be easily connected to the brace at regular time points for performing the prescribed exercises. The patient can himself connect the portable and compact brace provided with the motor, and as a consequence does not need to go to a hospital or clinic for fixing the brace and performing its prescribed exercises. Also the connection enables to closely connect the motor unit to the brace. The resulting rehabilitation device is compact and is not hindering the patient. Also, the brace is not cumbersome at all, and a patient is not hindered when using the motorized brace or when carrying the brace without the motor. Another advantage is the fact that the brace is portable by a patient.

The motor unit can be mechanically connected to the brace through several ways. One type of connection involves a frictional engagement of the motor unit in the connector elements. For instance, the motor can be provided with intrusions, which correspond to protrusions provided on the hinge of the brace. Intrusion may comprise openings in the motor units, wherein a pin provided in the hinge of the brace fit. Connection may also involve the use of clips, brackets, hooks, knobs, or the like. Also usable are cogwheels, releasable fastening means, or even by magnetic connections. In a particularly preferred embodiment, the connector elements **7** consist of a plate-like structure and a pin structure on the hinge as represented on figure 2 and 4. The motor unit is connected to said structures by means of bolts.

In another aspect, the present invention relates to a method for the rehabilitation of an injured limb whereby the above described rehabilitation device is used. Said method according to the invention comprises the steps of:
a) providing a rehabilitation device according to the invention,
b) determining the intended series of movements for said injured limb;
c) designing a software program for imposing the intended series of movements for said injured limb and for registering movements performed by said limb;
d) loading said software program on the motor unit of the rehabilitation device by the above-described method;
e) positioning the rehabilitation device on said injured limb,
f) imposing a movement on said injured limb by activating the motor unit of said brace, and
g) registering the movement performed by said limb.

In function of the injury, the patient's treatment plan and its pain tolerance a physician determines the appropriate series of movement exercises. Based thereon, a suitable software program is designed which is loaded on the motor unit of the brace according to the above-described method. Information of all movements to be performed over a certain specified period, i.e. the "schedule of movement exercises" is stored.

A patient may decide when he performs the movements. At that time, the brace comprising the motor unit is fitted to the injured limb every time a patient wishes to impose a well-defined movement on the injured limb. Preferably, the rehabilitation device is worn for intervals of several hours, and execution of the prescribed exercises is spread over several days, weeks or months, in function of the patient's rehabilitation program.

The brace is preferably attached to the patient's arm on upper and lower side of the elbow joint, or to a patient's leg, on upper and lower side of the knee joint. The brace is first positioned such that the first brace portion **1** is located on a first portion of the patient's limb on a first side of the joint and the second brace portion **2** is connected on a second portion of the patient's limb on the second side of the joint. The straps **8** are adjusted to secure the brace to the patient's limb.

An important characteristic of the method is the fact that every movement performed by the patient is registered. This is practically done by connecting the motor unit of said rehabilitation device, and in particular the control panel of the motor unit, to PC equipment and determining the registered performed movements, e.g. by visualization of the performed movements and motion data registered on a computer screen. As mentioned above, the control panel of the motor unit keeps a record of each performed exercise. Upon connection of the control panel of the motor to suitable PC equipment, a physician can control whether a patient is complying or has complied with the prescribed exercise regime. This enables a physician at any time in the treatment to investigate which movements have already been performed by the patient, and which movements still need to be performed in order to comply with the complete schedule of prescribed movements. It further permits the physician to evaluate the patient's progress in regard to the prescribed exercises, or to intervene and adapt the exercises in function of a patient's progress.

Preferably, in order to obtain full rehabilitation the steps e) and f) of the method are repeated until all intended, i.e. prescribed, movements have been performed. However, the prescribed schedule of movement exercises may also be adapted during the rehabilitation treatment. Since all movements performed by the patient are registered and can be controlled by a physician, the latter may decide to adapt the exercises to be performed in function of the patient speed of rehabilitation. For instance, therapy can consist of gradually increasing the force applied.

Since the rehabilitation device is easy to apply and to carry by a patient, a patient can perform all prescribed exercises at home, and does not need to go to a hospital or rehabilitation center. As it will be understood from the disclosure, a patient may also wear the rehabilitation device without the motor unit. In this case, the orthotic device according to the invention is meant for immobilizing and supporting an injured limb during the healing process.

The device and method according to the invention are particularly suitable in the field of paramedical and orthotic applications. In particular the invention can be used for the rehabilitation of all kind of injuries, especially of arm and leg injuries, and in the rehabilitation of the elbow or knee and hand or foot joints motions.

It will be appreciated by those skilled in the art that this invention is not limited to the particular embodiments disclosed, but it is intended to cover modifications within the spirit and scope of the present invention as defined by the appended claims.

## Claims

1. An orthotic device, for example a brace able to partly embrace and support a body part comprising an articulation, said device being connectable to a detachable motor unit and comprising
- a first device portion for supporting the portion of said body part provided on the upper side of a joint in said body part,
- a second device portion for supporting the portion of a body part comprising an articulation provided on the lower side of said joint in the body part,
- a hinge for connecting said first device portion with said second device portion, said hinge being provided with connector elements for temporarily attaching a detachable motor unit able to control the hinge,
whereby said connector elements are provided at the joint of the body part for temporarily attaching a detachable motor unit.

2. Brace according to claim 1, whereby said brace is fabricated to fit the limb anatomy of an individual person.

3. Brace according to claim 1 or 2, whereby said first and said second brace portion are provided with fastening means.

4. Brace according to any of claim 1 to 3, whereby the limb is a leg or an arm.

5. A motor unit being temporarily attachable to the brace according to any of claims 1 to 4 by means of connector elements provided on the hinge of said brace, said detachable motor unit being able to control the hinge of said brace.

6. A motor unit according to claim 5, whereby said motor unit is connectable to a control panel, said control panel being connectable to PC equipment and to a power-supplier.

7. A motor unit according to claim 5 or 6, whereby said motor unit is provided with a cable with plug for connecting said motor unit to a power-supplier.

8. A motor unit according to any of claims 5 to 7, whereby said motor unit is programmable for imposing movements to a limb.

9. A motor unit according to any of claim 5 to 8, whereby said motor unit is programmable for registering the movements performed by a limb.

10. A method for programming a motor unit according to any of claims 5 to 9, comprising the steps of connecting said motor unit with PC equipment via said control panel and loading a software program on said motor unit.

11. A software program for programming a motor unit according to any of claims 5 to 9.

12. A software program according to claim 11, whereby said program comprises a program for imposing movements to a limb and/or for registering the movements performed by said limb.

13. A rehabilitation device comprising a brace according to any of claims 1 to 4, and a motor unit according to any of claims 5 to 9, said motor unit being temporarily attached to said brace by means of the connector elements provided on the hinge of said brace.

14. A method for the rehabilitation of an injured limb comprising the steps of:
a) providing a rehabilitation device according to claim 13,
b) determining the intended series of movements for said injured limb;
c) designing a software program for imposing the intended series of movements for said injured limb and for registering movements performed by said limb;
d) loading said software program on the motor unit of the rehabilitation device by the method according to claim 10;
e) fitting the rehabilitation device on said injured limb,
f) imposing a movement on said injured limb by activating the motor unit of said brace, and
g) registering the movement performed by said limb.

15. A method according to claim 14 whereby steps e) to g) are repeated until all intended series of movements have been performed.

16. A method according to claim 14 or 15, further comprising the step of controlling the registered movements by connecting the motor unit of said rehabilitation device to PC equipment and determining the registered performed movements.

17. Use of the brace according to any of claim 1 to 4, or the rehabilitation device according to claim 13 in rehabilitation treatments.
